# EUROPEAN PATENT APPLICATION

(11) **EP 4 813 506 A1**
(43) Date of publication of application: **30.09.2026**
(21) Application number: 24907529.2
(22) Date of filing: 20.12.2024
(51) Int. Cl.: B01D 61/36, B01J 20/02, B01J 20/34, C07C 29/76, C07C 31/04, C07C 31/08

(54) **METHOD FOR PRODUCING SEPARATION/CONCENTRATION PRODUCT OF VOLATILE SUBSTANCE, AND SYSTEM FOR PRODUCING SEPARATION/CONCENTRATION PRODUCT OF VOLATILE SUBSTANCE**

(30) Priority: 22.12.2023 JP 2023216419
(71) Applicant: National Institute of Advanced Industrial Science and Technology, Chiyoda-ku Tokyo 100-8921 (JP)
(72) Inventor: KAWAMOTO, Tohru, Tsukuba-shi, Ibaraki 305-8560 (JP); MINAMI, Kimitaka, Tsukuba-shi, Ibaraki 305-8560 (JP); TAKAHASHI, Akira, Tsukuba-shi, Ibaraki 305-8560 (JP); SHUDO, Yuta, Tsukuba-shi, Ibaraki 305-8560 (JP); YOSHIOKA, Tomohisa, Kobe-shi, Hyogo 657-8501 (JP); MATSUYAMA, Hideto, Kobe-shi, Hyogo 657-8501 (JP); LI, Zhan, Kobe-shi, Hyogo 657-8501 (JP)
(74) Representative: Goddar, Heinz J.
(86) International application number: PCT/JP2024/045099
(87) International publication number: WO 2025/135145

(57) **Abstract**

[Problem]

The purpose of the present invention is to provide a method and a system which are for producing a separation/concentration product of a volatile substance, and which are capable of concentrating the volatile substance to a high concentration and suppressing energy consumption and material consumption by using a small-sized apparatus.

[Solution]

This method for producing a separation/concentration product of a volatile substance is for concentrating an aqueous solution containing a volatile substance or for isolating the volatile substance, and is characterized by comprising the following steps. The method is characterized in that the volatile substance is selectively adsorbed in the following adsorption step and/or the volatile substance is selectively desorbed in the following desorption step. Step 1: Vaporization step Step 2: Adsorption step Step 3: Desorption step.

## Description

### TECHNICAL FIELD OF THE INVENTION

The present invention relates to a method and a system for concentrating and separating a volatile substance from an aqueous solution containing the volatile substance to produce a high-concentration aqueous solution of the volatile substance or the highly-purified volatile substance.

### BACKGROUND OF THE INVENTION

Treatment of aqueous solutions of volatile and water-soluble substances such as ammonia and methanol or recovering such the substances has been a long-standing problem. When the treatment, namely, detoxification, is the purpose, methods such as a standard activated sludge process, combustion method, and catalytic decomposition method have been used. Since the standard activated sludge process utilizes biological activity, it is required that the aqueous solution contains carbon and nitrogen in an appropriate ratio. Thus, utilization of the method is difficult when the solution is mostly ammonia or mostly methanol. In the combustion method or catalytic decomposition method, on the other hand, the substances are to be vaporized first before treatment and water is also to be vaporized at the same time, which requires a lot of energy.

Meanwhile, there also have been efforts to recover and recycle such volatile substances to be utilized as valuable resources through concentration or purification. Examples include a utilization of a distillation method. However, when using water-soluble substances such as ammonia and methanol whose boiling points are not so different from that of water, multi-stage processing is required, which still has problems such as having larger apparatus and significant energy loss.

The most well-known method for concentrating ammonia water, which is a volatile substance, is an ammonia stripping method. The method includes adjusting pH of an aqueous solution containing ammonia to be alkalized, increasing the temperature, and bringing the solution into contact with a gas phase to evaporate ammonia preferentially. For example, by bringing such the evaporated ammonia into contact with an aqueous sulfuric acid solution, ammonium sulfate water can be produced, and with further evaporation to dryness, ammonium sulfate can be obtained.

Alternatively, a membrane distillation method can also be used for concentrating volatile substances. Such the method utilizes a hydrophobic separation membrane, where an aqueous solution comes into contact with one side of the membrane and the volatile substance is volatized to the other side to be concentrated. For example, Non-Patent Document 1 has reported that, in a case of ammonia water, a 4% aqueous solution can be concentrated to 20% or more.

As another method, a liquid-phase concentration method using an adsorbent has also been considered. Patent Document 1, for example, has described a method in which a metal cyano complex is used as an adsorbent such that ammonium ions are adsorbed in a liquid phase by ion exchange from a system containing coexisting substances, and then the ammonium chloride aqueous solution is recovered by washing the adsorbent with a potassium chloride aqueous solution.

Furthermore, methods combining membrane distillation and adsorption have also been proposed (Patent Documents 2, 3, and 4).

### PRIOR ARTS

### PATENT DOCUMENTS

[Patent Document 1] Japanese Patent No. 6970406 Specification
[Patent Document 2] Japanese Patent Application Laid-Open Publication No. 2019-107646 (JP-A-2019-107646)
[Patent Document 3] National Publication of International Patent Application No. 2021-513457 (JP-T-2021-513457)
[Patent Document 4] National Publication of International Patent Application No. 2015-535301 (JP-T-2015-535301)

### NON-PATENT DOCUMENT

[Non-Patent Document 1] Concentration and resource utilization technology of ammonia in water, Hideto Matsuyama, The Second Nitrogen Cycle Symposium "Global Challenges and Initiatives in Japan regarding Nitrogen Cycle", https:www.n-cycle.jp/events/symposium20221121/Hideto Matsuyama's presentation material p.11

### SUMMARY OF THE INVENTION

### (PROBLEMS TO BE SOLVED BY THE INVENTION)

However, the above-mentioned methods have advantages and disadvantages. In the ammonia stripping method, all the water before concentration is to be heated, which requires a lot of energy. Also, the size of the apparatus is to be large, which is another problem. In the membrane distillation method shown in Non-Patent Document 1, although volatile substances can be vaporized without heating, thereby consuming less energy compared to the ammonia stripping method, energy for vaporization heat associated with water vaporization is still necessary. In particular, when concentration of the volatile substance in an aqueous solution is low, an amount of water vaporized relative to the target volatile substance increases, which increases the energy required and makes it difficult to increase the concentration after concentration. Furthermore, for concentrating substances such as salt or pure ammonia to high concentration, a treatment such as evaporation to dryness is required following the ammonia stripping or membrane distillation, which requires even more energy. The method using adsorbent described in Patent Document 1 has a few problems, including the need for potassium chloride as a chemical agent, a requirement to further solidify the concentrated liquid using distillation or other methods to obtain a solid salt, which requires energy, and inability to obtain ammonia, which is not a salt. Also, the method combining the membrane distillation and adsorption is for removing impurities through adsorption process as a pretreatment or posttreatment for membrane distillation, and thus is not to solve the above-mentioned problems of energy consumption.

Thus, to efficiently concentrate volatile substances, a method in which energy consumption is suppressed by suppressing water vaporization is required. In particular, even when concentration of a volatile substance in raw water is low, a material that can selectively adsorb or vaporize the volatile substance is required. In view of such points, it is an object of the present invention to provide a method and a system which are for producing a separation/concentration product of a volatile substance, and which are capable of concentrating the volatile substance to high concentration and suppressing energy consumption and material consumption by using a small-sized apparatus.

### (MEANS FOR SOLVING PROBLEMS)

After various studies, the inventors found a way to solve such problems by combining a gas sweep membrane distillation method with an adsorbent that selectively adsorbs and desorbs volatile substances.

The present invention was achieved based on such findings and the present invention provides the following means.
(1) A method for producing a separation/concentration product of a volatile substance is a method for concentrating an aqueous solution containing a volatile substance or for isolating the volatile substance, including steps below, characterized in that the volatile substance is selectively adsorbed in an adsorption step below and/or selectively desorbed in a desorption step below;
   Step 1: A step for obtaining a mixed gas by using a membrane that is impermeable to water but permeable to gas for separation, with one side of the membrane being a liquid phase side with which the aqueous solution containing the volatile substance comes into contact, and the other side of the membrane being a gas phase side, and allowing the vaporized volatile substance and water vapor to permeate to the gas phase side (Vaporization Step),
   Step 2: A step for adsorbing the volatile substance by bringing the gas obtained in Step 1 containing the volatile substance into contact with an adsorbent that can adsorb the volatile substance (Adsorption Step), and
   Step 3: A step for desorbing the volatile substance from the adsorbent, obtained in Step 2, that has adsorbed the volatile substance (Desorption Step).
(2) The method for producing a separation/concentration product of a volatile substance according to (1) is characterized in that the mixed gas obtained in Step 1 is brought into contact with the adsorbent in Step 2 and the gas after contact is reintroduced to the gas phase side of the membrane in Step 1 so as to suppress vaporization of water.
(3) The method for producing a separation/concentration product of a volatile substance according to (1) is characterized in that the volatile substance is ammonia.
(4) The method for producing a separation/concentration product of a volatile substance according to (1) is characterized in that the volatile substance is methanol.
(5) The method for producing a separation/concentration product of a volatile substance according to (1) is characterized in that the adsorbent is a Prussian blue derivative represented by the following general formula (1).

   AₓM[M'(CN)₆]_{y} ·zH₂O ··· (1)

   wherein, x represents a number from 0 to 3, y represents a number from 0.1 to 1.5, z represents a number from 0 to 6, A represents a cation representing at least one or a combination of two or more selected from a group consisting of hydrogen, ammonium cations, alkali metal ions, and alkaline earth metal ions, M and M' are respectively and independently selected, M represents a cation representing at least one or a combination of two or more selected from a group consisting of atoms with atomic numbers from 3 to 83 (excluding ammonium cations, alkali metal ions, and alkaline earth metal ions), and M' represents a cation representing at least one or a combination of two or more selected from a group consisting of atoms with atomic numbers from 3 to 83 (excluding ammonium cations, alkali metal ions, and alkaline earth metal ions).
(6) The method for producing a separation/concentration product of a volatile substance according to (1) is characterized in that the volatile substance is desorbed in Desorption Step by heating the adsorbent.
(7) The method for producing a separation/concentration product of a volatile substance according to (1) is characterized in that, when heating the adsorbent in Desorption Step, temperature is adjusted in two stages, low and high, so that the volatile substance can be selectively desorbed at the high temperature.
(8) A system for producing a separation/concentration product of a volatile substance is a system for concentrating an aqueous solution containing a volatile substance or for isolating the volatile substance in which adsorption means below selectively adsorbs the volatile substance and/or desorption means below selectively desorbs the volatile substance, and is characterized by including means below:
   Means 1: Means for obtaining a mixed gas by using a membrane that is impermeable to water but permeable to gas for separation, with one side of the membrane being a liquid phase side with which the aqueous solution containing the volatile substance comes into contact, and the other side of the membrane being a gas phase side, and allowing the vaporized volatile substance and water vapor to permeate to the gas phase side (Vaporization Means),
   Means 2: Means for adsorbing the volatile substance by bringing the gas obtained by Means 1 containing the volatile substance into contact with an adsorbent that can adsorb the volatile substance (Adsorption Means), and
   Means 3: Means for desorbing the volatile substance from the adsorbent, obtained by Means 2, that adsorbed the volatile substance (Desorption Means).
(9) The system for producing a separation/concentration product of a volatile substance according to (8) is characterized in that the adsorbent is a Prussian blue derivative represented by the following general formula (1).

   AₓM[M'(CN)₆]_{y} ·zH₂O ··· (1)

   wherein, x represents a number from 0 to 3, y represents a number from 0.1 to 1.5, z is a number from 0 to 6, A represents a cation representing at least one or a combination of two or more selected from a group consisting of hydrogen, ammonium cations, alkali metal ions, and alkaline earth metal ions, M and M' are respectively and independently selected, M represents a cation representing at least one or a combination of two or more selected from a group consisting of atoms with atomic numbers from 3 to 83 (excluding ammonium cations, alkali metal ions, and alkaline earth metal ions), and M' represents a cation representing at least one or a combination of two or more selected from a group consisting of atoms with atomic numbers from 3 to 83 (excluding ammonium cations, alkali metal ions, and alkaline earth metal ions).
(10) The system for producing a separation/concentration product of a volatile substance according to (8) further includes Means 2.5 (Return Means) provided between Means 2 and 3, for returning the gas that is not adsorbed by the adsorbent to the gas phase side of the membrane, thereby enabling continuous vaporization via the membrane.
(11) The system for producing a separation/concentration product of a volatile substance according to (8) is characterized in that, by Means 3, while the adsorbent obtained by Means 2, which has adsorbed the volatile substance, is removed from a reaction system and the volatile substance is desorbed through a predetermined method, a new adsorbent is supplied to the part from which the adsorbent was removed, thereby maintaining a continuous cycle of vaporization of the volatile substance.

### (EFFECTS OF THE INVENTION)

The present invention enables concentration or purification of a volatile substance from raw water containing such the volatile substance. At this time, a small-sized apparatus can be used, energy consumption associated with water vaporization can be reduced, and components can be reused, thereby keeping material costs low. Furthermore, according to the present invention, even when the raw water has a low concentration, the volatile substance can be concentrated to high concentration.

### BRIEF DESCRIPTION OF DRAWINGS

FIG. 1 is a schematic view illustrating apparatus (system) for separation/concentration of ammonia.
FIG. 2 is a photograph (left) and a schematic view (right) showing a triple-tube nozzle used in manufacturing a hollow fiber membrane.
FIG. 3 is a photograph substituting a drawing, taken by an electron microscope from a side view, of contacting forms of water and ammonia water on the hollow fiber membrane.
FIG. 4 is a graph showing experimental results of separating ammonia and water using a separation membrane.
FIG. 5 is a graph showing desorption profiles of water and ammonia from Prussian blue as temperature rises.
FIG. 6 is a graph showing a change in methanol concentration in circulating fluid.
FIG. 7 is a graph showing flow of methanol solution and water in relation to an amount of methanol supplied.
FIG. 8 is an infrared spectroscopic diagram of a manganese-cobalt cyano complex that has adsorbed under methanol atmosphere.
FIG. 9 is an infrared spectroscopic diagram when the manganese-cobalt cyano complex that has absorbed methanol is heated.
FIG. 10 is a structural view showing schematically a crystal structure of a Prussian blue derivative.

### DESCRIPTION OF SOME EMBODIMENTS

Hereinafter, some embodiments of the present invention (hereinafter, sometimes referred to as "the present embodiments") will be described. The descriptions below are for illustrative purposes only and do not limit the scope of the present invention.

Note that "- (to)" indicating a numerical range includes the numerical values therebefore and thereafter as lower and upper limits, respectively.

Volatile substances to be concentrated/separated in the present invention (hereinafter, referred to as "target volatile substance") may be any substance that dissolves in water, and has a constant vapor pressure at room temperature (23°C), and at least a portion thereof evaporates from water at room temperature (23°C). Preferable examples are compounds with relatively small molecular weights such as ammonia, methanol, and ethanol. Examples include ammonia, amines with 1 to 3 carbon atoms, alcohols with 1 to 3 carbon atoms, aldehydes with 1 to 3 carbon atoms, ketones with 3 to 5 carbon atoms, ethers with 2 to 4 carbon atoms, esters with 3 to 5 carbon atoms, and carbonates with 3 to 5 carbon atoms.

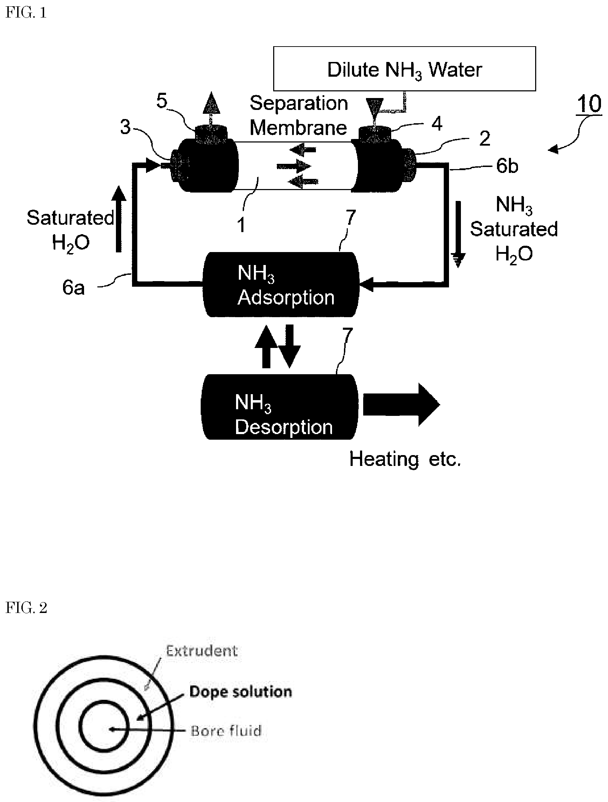

Apparatus that implements the present invention (FIG. 1) includes, broadly, a membrane distillation portion (separator) 1 provided with a separation membrane, an adsorption portion (adsorbent) 7, and circulation connection portions 6a and 6b. Reference numeral 2 denotes an outlet for a gas containing a target volatile substance (such as ammonia), and reference numeral 3 denotes an inlet for the gas after adsorption of the target volatile substance (such as ammonia). Reference numeral 4 denotes an inlet for water containing the target volatile substance (such as dilute ammonia water), and reference numeral 5 denotes an outlet for the water containing the target volatile substance (such as dilute ammonia water). It is a purpose of the membrane distillation portion 1 to vaporize the target volatile substance from the water containing the target volatile substance. However, some water vapor may also vaporize. It is a purpose of the adsorption portion 7 to selectively adsorb the target volatile substance vaporized in the membrane distillation portion 1 and then to selectively desorb and recover the target volatile substance. Selectivity for adsorption and desorption may be both or just one. It is a purpose of the circulation connection portions 6a and 6b to create a circulation structure that allows the vaporized target volatile substance, water vapor, etc. to flow between the membrane distillation portion and the adsorption portion and to return to the membrane distillation portion. The key point here is that, when the target volatile substance and other substances, including water vapor, vaporized in the membrane distillation portion enter the adsorption portion via a circulation flow portion, the target volatile substance is selectively adsorbed, thereby reducing concentration of the target volatile substance in the gas that is to be returned to the membrane distillation portion. Since the water vapor and other substances remain as gas without being adsorbed, vaporization progresses in the membrane distillation portion, thereby increasing concentration and eventually reaching the partial pressure equal to the vapor pressure. At this point, vaporization in the membrane distillation portion stops and no further vaporization occurs. On the other hand, since the target volatile substance is recovered in the adsorption portion, the concentration thereof in the circulating gas is maintained at a low level. As a result, only the target volatile substance is continuously vaporized in the membrane distillation portion. From the above, it is possible to suppress vaporization of other substances, including water vapor, allowing only the target volatile substance to vaporize, thereby avoiding energy loss associated with water vapor vaporization. Furthermore, since the target volatile substance is selectively adsorbed to and/or selectively desorbed from the adsorbent, the target volatile substance of high concentration can be recovered.

For the membrane distillation portion, a membrane for membrane distillation that does not allow liquid water to permeate but allows only vaporized gaseous substances to permeate is used. The membrane for membrane distillation separates a liquid flow portion from a gas flow portion. By passing water containing a volatile substance into the liquid flow portion and maintaining a constant temperature, the volatile substance in the liquid vaporizes and permeates, together with water vapor, through to the gas flow portion via the membrane. There are no limitations on a form and material of the membrane for membrane distillation as long as functions described herein are fulfilled. Examples of the form includes hollow fiber and flat membranes. The material such as polypropylene (PP), polytetrafluoroethylene (PTFE), and polyvinylidene fluoride (PVDF: polyvinylidene difluoride) can be used, and polyvinylidene fluoride (PVDF) is particularly preferred due to good processability etc. Since gas sweep membrane distillation method (SGMD) is used in embodiments of the present invention, it is preferable to use a hollow fiber membrane that is suitable for such the method. Dimensions and other specifications of the hollow fiber membrane can be determined as appropriate, and examples are: an outer diameter of 0.5 to 1.2 mm, an inner diameter of 0.3 to 0.8 mm, a membrane thickness of 0.07 to 0.3 mm, a porosity of 30 to 60%, an average pore diameter of 10 to 50 nm, a water contact angle of 100° to 140°, and an ammonia water contact angle of 100° to 140°. Although there are no particular limitations, molecular weight of PVDF used here is preferably, in Mw, around 400,000 to 1,000,000, more preferably around 500,000 to 8800,000, and even more preferably around 600,000 to 700,000.

Although there are no limitations on a method for manufacturing the hollow fiber membrane and any method used in such the field may be used, it is preferable to employ a thermally induced phase separation (TIPS) process using a triple-tube nozzle shown in FIG. 2. Diethyl phthalate, PEG400, GTA (glycerol triacetate), or the like can be used for bore fluid of the triple-tube nozzle. For a dope solution, which is to be an intermediate layer, a solution of polyvinylidene fluoride dissolved in diphenyl carbonate or DMP, for example, can be used. Dissolution concentration is preferably 10 to 50 mass% and more preferably 15 to 35 mass%. For extrudent, which is to be an outermost layer, tributyl acetyl citrate, PEG400, DOP (dioctyl phthalate), etc., can be used. Temperature for extrusion may be, although not particularly limited, 150 to 300°C. The extruded hollow fiber membrane is placed into water (5 to 20°C) and rapidly cooled to maintain its shape.

Details of the method of manufacturing the hollow fiber membrane (TIPS) and the phase separation of ammonia by SGMD using the hollow fiber membrane are disclosed in the following reference: Zhan Li, Pengfei Zhang, Kecheng Guan, Ralph Rolly Gonzales, Toru Ishigami, Ming Xue c, Tomohisa Yoshioka, Hideto Matusyama, Process Safety and Environmental Protection 171 (2023) 555-560, "An experimental study on recovering and concentrating ammonia by sweep gas membrane distillation".

There are no particular limitations on a form of the liquid flow portion inside the membrane distillation portion as long as water containing a volatile substance comes into contact with the membrane for membrane distillation. However, since it is generally required to process a large amount of water relative to a membrane surface area, it is common that the liquid flow portion is a flow-through system with an inlet and an outlet. Material thereof is to fulfill durability such as water resistance and heat resistance, and polymer materials such as polypropylene and polyvinyl chloride as well as metals such as stainless steel can be used.

The gas flow portion is to have a form, as described below, in which the gas passed through the adsorption portion comes into contact with the membrane again. The form and material thereof are to fulfill durability such as water resistance and heat resistance, and the flow-through system may be commonly employed as the form. However, the gas flow portion is to be filled with volatile substance gas of high concentration and it is necessary to pay attention particularly to the durability. For example, if the volatile substance is ammonia, materials lacking corrosion resistance, such as copper and aluminum, cannot be used. Thus, for example, ceramic piping or piping with inner surfaces coated with PTFE or the like can be used.

The adsorption portion includes an adsorbent that selectively adsorbs the target volatile substance, and brings the gas vaporized in the membrane distillation portion into contact with the adsorbent, thereby recovering the volatile substance. Material for the adsorbent is selected depending on the target volatile substance, and it is required that the material can adsorb the volatile substance selectively and also can desorb the adsorbed volatile substance by some means. In this context, "adsorb selectively" means that, when comparing the target volatile substance with water vapor, the volatile substance is preferentially adsorbed. "Adsorbed preferentially" can be expressed by a selectivity coefficient (based on molar ratio) in the following formula for a case in which gas of a volatile substance and water vapor in a ratio of x : y is brought into contact with an adsorbent. $\begin{matrix}Selectivity Coefficient = \left(Amount of Adsorbed Volatile Substance/x\right) / \\ \left(Amount of Adsorbed Water Vapor\right) / y \end{matrix}$

In the present invention, the selectivity coefficient is preferably 1 or more, more preferably 2 or more, furthermore preferably 5 or more, and even more preferably 10 or more. Although the upper limit is not particularly limited, it is practical to set to 1000 or less.

Materials such as a metal cyano complex, zeolite, and metal-organic framework (MOF) can be used as the adsorbent, though the materials are not limited to the above as long as the above-mentioned requirements are fulfilled. In particular, the metal cyano complex is known for an ability to selectively adsorb ammonia and methanol and can be used for separation/concentration thereof.

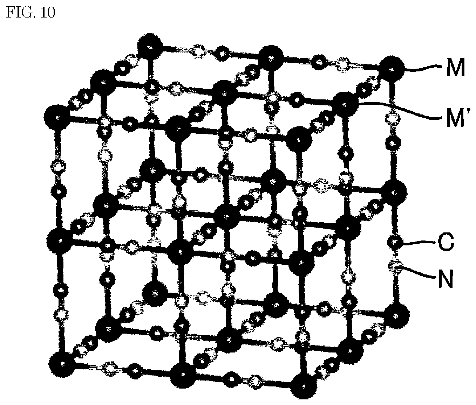

The metal cyano complex in the present embodiment (hereinafter, a metal cyano complex, including an iron-iron metal cyano complex (Prussian blue), in which the metal M and/or metal M' is substituted with a metal other than iron may be referred to as a Prussian blue derivative) is a type of a porous coordination polymer, and includes a metal ion (a positively charged cation) and a cyano group (CN⁻ of a negatively charged anion), which is a type of ligand crosslinking the metal ion (see FIG. 10). Metal cyano complexes are a series of compounds called the metal cyano complexes that structurally include hexacyano metal ions. The metal cyano complex is provided with a nano-porous structure that allows to take in the target gas. The size of the nano-porous structure, or pore size, is 0.3 to 0.6 nm. The metal cyano complex is assembled with the regularly repeated nano-porous structures. Thus, the metal cyano complex has a large surface area and can efficiently adsorb and desorb ammonia species with high selectivity.

The metal cyano complex in the present embodiment has a face-centered cubic structure, which is a typical crystal structure, as shown in FIG. 10, and is represented by the following general formula (1):

AₓM[M'(CN)₆]_{y} ·zH₂O ··· (1)

wherein, x represents 0 to 3, y represents 0.1 to 1.5, and z represents 0 to 6. A represents one or more cations of hydrogen, ammonium, alkali metals, and alkaline earth metals. M and M' are independent of each other and represent one or more cations of atoms with atomic numbers 3 to 83. However, M and M' are not ammonium cations, alkali metal cations, or alkaline earth metal cations.

A is one or more cations of alkali metals of hydrogen, ammonium, lithium, sodium, potassium, rubidium, cesium, and francium, as well as alkaline earth metals of magnesium, calcium, strontium, barium, and radium. A may contain a mixture of two or more cations, and x is a value that maintains an overall charge balance of the metal cyano complex.

Examples of M include one or more metallic cations of vanadium, chromium, manganese, iron, ruthenium, cobalt, rhodium, nickel, palladium, platinum, copper, silver, zinc, indium, lanthanum, europium, gadolinium, and lutetium. M may be a mixture of two or more cations and is present in the metal cyano complex to maintain the overall charge balance of the metal cyano complex. Examples of M' include one or more metallic cations of vanadium, chromium, molybdenum, tungsten, manganese, iron, ruthenium, cobalt, nickel, platinum, and copper. From a viewpoint of stability of cyanide in PB derivatives, M' is preferably iron or cobalt.

By combining M and M', characteristics of the adsorbent, such as adsorption capacity, adsorption rate, selectivity, and material stability, can be controlled. An appropriate combination of M and M' varies depending on the target volatile substance. For example, if the target is ammonia, following combinations of which adsorption properties to ammonia are known can be used: Fe³⁺ for M and Fe²⁺ for M', Cu²⁺ for M and Fe²⁺ for M', Co²⁺ for M and Fe³⁺ for M', Ni²⁺ for M and Fe³⁺ for M', Ni²⁺ for M and Fe²⁺ for M', and Co²⁺ for M and Co³⁺ for M'; and if the target is methanol, following combinations of which adsorption properties to methanol are known can be used: Mn²⁺ for M and Co²⁺ for M', Mn²⁺ for M and Fe²⁺ for M', and Mn²⁺ for M and Fe³⁺ for M'.

The adsorbent provided in the adsorption portion may be in any form as long as the adsorbent and the vaporized volatile substance can come into contact with each other appropriately. For example, granular materials molded into granules, a support in which the adsorbent is supported on a base material, or powder can be used. There are no particular limitations on a method for molding granular materials as long as the vaporized volatile substance can be adsorbed. For example, methods described in specifications of Japanese Patent No. 6918323 and Japanese Patent Laid-open Publication No. 2014-077720 can be used. Also, the granule materials may contain other substances, such as polymers that act as binders or flocculants to improve efficiency of the manufacturing process. Similarly, there are no particular limitations on a method for molding the support as long as the vaporized volatile substance can be appropriately adsorbed. For example, a method described in specifications of Japanese Patent No.6531889 can be used.

Existing predetermined methods can also be used to desorb the volatile substance from the adsorption portion. For example, when the volatile substance is ammonia and the adsorbent is a metal cyano complex, methods such as a method of contacting with acid and water, a heating method, or a ultraviolet irradiation method as described in specifications of Japanese Patent No. 6345774, a method of contacting with carbon oxide and water as described in Japanese Patent No. 7093582, a method utilizing humidification as described in specifications of Japanese Patent No. 7209994, and a method of contacting with ammonium bicarbonate as described in Japanese Patent Laid-open Publication No. 2022-189786 can be used. Also, although details will be described below, the volatile substance may be isolated by performing desorption by heating and utilizing the difference in desorption temperatures between water and the volatile substance.

However, the process is to be divided into a step of recovering the volatile substance in the adsorption portion and the step of desorption. For example, the connection between the membrane distillation portion and the adsorption portion may be disconnected , the adsorption portion may be removed, and the above-mentioned desorption process can be performed. Alternatively, multiple adsorption portions may be prepared, one connected to the membrane distillation portion for the adsorption process and another disconnected for the desorption process, thereby continuously introducing new adsorbent for adsorption and desorption.

It is preferable that the temperature of the volatile substance desorbing from the adsorbent is different from that of coexisting water. Specifically, the desorption temperature of water is preferably 40 to 120°C, and even more preferably 50 to 100°C. The desorption temperature of the volatile substance is preferably 100 to 200°C, and even more preferably 110 to 180°C. The difference in the desorption temperature between water and the volatile substance is, at a peak top in a desorption profile, preferably 10°C or more, more preferably 25°C or more, and even more preferably 50°C or more. By using such the difference in the desorption temperatures, the volatile substance can be selectively separated, concentrated, and recovered. Unless otherwise specified, the peak temperature of the desorbed substance refers to a peak measured using TG-MS at a heating rate of 1°C / 1 minute. Such the desorption process using the desorption temperature difference will be referred to as a form of selective desorption. Alternatively, including the above form, even when not using temperature but when desorbing by washing and releasing with time delay, or when desorbing by pressure, for example, the desorption process can use separation by pressure or time, and thus the process can be referred to as a form of selective desorption. That is, the process of separating a target substance (a volatile substance) from water under such certain conditions during the desorption step is called "selective desorption".

It is a purpose of the circulation connection portion to connect the membrane distillation portion with the adsorption portion, allowing gas to flow. It is preferable to use material commonly used in the field of the present invention, and it is preferable to select the material considering corrosion resistance to volatile substances and cost, etc. When the volatile substance is alcohol, metal-made or resin-made pipes with relatively low corrosion resistance can be used as appropriate. When the volatile substance is ammonia, it is preferable to select the material for the piping taking corrosion resistance into consideration more carefully. For example, piping made of ceramics or piping of which inner surfaces are coated with PTFE can be used.

According to a preferred embodiment of the present invention, a system for producing a separation/concentration product of a volatile substance is a system for concentrating an aqueous solution containing a volatile substance or for isolating the volatile substance. In particular, adsorption means described below is for selectively adsorbing the volatile substance, and/or desorption means described below is for selectively desorbing the volatile substance, and is characterized by including means below, although Means 2.5 is optional.
1. Means for obtaining a mixed gas by using a membrane that is impermeable to water but permeable to gas for separation, with one side of the membrane being a liquid phase side with which the aqueous solution containing the volatile substance comes into contact and the other side of the membrane being a gas phase side, and allowing the vaporized volatile substance and water vapor to permeate to the gas phase side (Vaporization Means),
2. Means for adsorbing the volatile substance by bringing the mixed gas obtained by Means 1 containing the volatile substance into contact with an adsorbent that can adsorb the volatile substance (Adsorption Means),
2.5 Means for maintaining a cycle in which the gas that is not adsorbed by the adsorbent is returned to the gas phase side of the membrane, thereby enabling continuous vaporization via the membrane (Returning Means), and
3. Means for removing the adsorbent that has adsorbed the volatile substance obtained by Means 2 or Means 2.5 from a reaction system and desorbing the volatile substance by a predetermined method such as heating, while supplying a new adsorbent to the part from which the adsorbent was removed to maintain a continuous cycle of vaporization , (Desorption Means).

### WORKING EXAMPLES

Hereinafter, the present invention will be described in more detail based on working examples. However, the present invention is not to be construed as being limited thereto.

### (Preparation Example 1)

### (Preparation of Copper-Iron Cyano Complex)

A granulated adsorbent of copper-iron cyano complex was prepared by the method described in Working Example 1 of Patent No. 6918323. Specific procedures are as follows.

### 1. Preparation of raw aqueous solution

As a raw aqueous solution, 7.77 moles of copper sulfate pentahydrate were dissolved in 9.25 kg of water to prepare 10 L of copper sulfate aqueous solution LC1. Furthermore, 4.24 moles of potassium ferrocyanide trihydrate were dissolved in 8.15 kg of water to prepare 9 L of potassium ferrocyanide aqueous solution LF1.

### 2. Synthesis of metal cyano complex slurry

LC1 was mixed with LF1 to prepare a copper-iron cyano complex slurry S1, with M = Cu²⁺ and M' = Fe²⁺.

### 3. Addition of flocculant precipitating agent

After adjusting pH of the slurry S1, 24g of a polymer flocculant precipitating agent that mainly includes a copolymer of acrylamide and dimethyl aminoethyl methacrylate with a molecular weight of approximately 4000000 was added to the slurry and stirred with a stirrer.

### 4. Manufacturing dried block

The slurry S1 was compressed using an electric hydraulic press (YS-1, manufactured by Hashida Kiko Co., Ltd.) to separate solid and liquid components, and dewatered cake C1 was prepared. The dewatered cake C1 was cut into small pieces about 3 cm square, and then dried in a shelf dryer at 40°C for 24 hours to prepare a dried block B1.

### 5, Crushing block into fine powder, addition of binder and crosslinking agent, and mixing

The dried block B1 was crushed using a cutter mixer (BLIXER6, manufactured by FMI Corporation) to obtain dried powder DP1. At this time, it was made sure that the temperature of the cutter mixer did not exceed 40°C.

Separately, a 10% by weight aqueous solution of polyvinyl alcohol was prepared as a binder, and titanium lactate ammonium salt was prepared as a crosslinking agent. Without removing P1 from the cutter mixer, the binder and crosslinking agent were added to P1 in that order. By operating the cutter mixer for one minute for stirring, wet powder WP1 in which the copper-iron cyano complex, the flocculant precipitating agent, binder, and crosslinking agent were mixed together was obtained.

### 6. Granulation and drying

The wet powder WP1 was fed into an extrusion granulator (F-5, manufactured by Dalton Corporation) to obtain a water-containing granulated adsorbent WA1. The water-containing granulated adsorbent WA1 was dried in the shelf dryer at 50°C for 24 hours to obtain a granulated adsorbent A1.

### (Production Example 1)

An ammonia separation membrane (hollow fiber membrane) was produced through the following procedure. Polyvinylidene fluoride (PVDF, Solef 6020, Mw = 670,000 to 700,000) made by Solvay Specialty Polymers Japan K.K. was used as the raw material. The hollow fiber membrane was produced by using a TIPS (thermally-induced phase separation) method (for details, see P. Zhang, S. Rajabzadeh, A. Venault, S. Wang, Q. Shen, Y. Jia, C. Fang, N. Kato, Y. Chang, H. Matsuyama, One-step entrapment of a ps-pegma amphiphilic copolymer on the outer surface of a hollow fiber membrane via tips process using triple-orifice spinneret, J. Membr. Sci. 638 (2021), 119712.; P. Zhang, W. Liu, S. Rajabzadeh, Y. Jia, Q. Shen, C. Fang, N. Kato, H. Matsuyama, Modification of Pvdf hollow fiber membrane by co-deposition of Pda/Mpc-co-aema for membrane distillation application with anti-fouling and anti-scaling properties, J. Membr. Sci. 636 (2021), 119596; and P. F. Zhang, C. J. Fang, S. Rajabzadeh, W. Y. Liu, Y. D. Jia, Q. Shen, L. Zhang, S. Y. Wang, N. Kato, H. Matsuyama, Effect of polymer molecular weight on structure and performance of Pvdf hollow fiber membranes prepared via tips process with co-extrusion of solvent using triple orifice spinneret, J. Membr. Sci. 620 (2021), 118854.). Firstly, PVDF was dissolved in DMP (dimethyl phthalate) at 200°C to prepare a homogeneous doped solution. The PVDF concentration was controlled to 24% by weight. The doped solution was then fed to a two-screw extrusion forming machine via a precisely-controlled conveying screw. The doped solution was co-extruded with PEG400 (polyethylene glycol, number average molecular weight 400) and fed to a cooling bath of 15°C water via a triple orifice. Here, PEG400 was allowed to flow through channels of inner and outermost layers, forming internal pores inside the hollow fiber membrane and a restricted surface of the hollow fiber membrane. The hollow fiber membrane was collected using a winding machine with a speed of 10 m/min. Next, the hollow fiber membrane was stored in fresh ethanol to wash off DMP (ethanol was changed twice a day). Five days later, the hollow fiber membrane was taken out and dried vertically at room temperature.

Conditions and parameters for manufacturing the PVDF hollow fiber membrane are as follows.

| Manufacturing Conditions | Parameters |
|---|---|
| PVDF concentration (mass%) | 24 |
| Mixing temperature (°C) | 200 |
| Screw speed (rpm) | 100 |
| Extrusion rate of doped solution (gmin⁻¹) | 7 |
| Flux of bore liquid (gmin⁻¹) | 3.5 |
| Flux of extrusion solvent (gmin⁻¹) | 2.5 |
| Air gap (cm) | 15 |
| Cooling bath temperature (°C) | 15 |
| Winding speed (m/min) | 12 |

Specifications of the hollow fiber membrane obtained as above are shown in the table below.

| Item | Parameters |
|---|---|
| Outer diameter | 0.9 mm |
| Inner diameter | 0.6 mm |
| Membrane thickness | 0.15 mm |
| Porosity | 42 to 46% |
| Average pore size | 18 nm |
| Water contact angle | 124° |
| 5% ammonia water contact angle | 124° |

Dimensions such as the outer diameter, inner diameter, and membrane thickness were measured by cutting the hollow fiber membrane crosswise with a fine cutter and observing the cross section with an electron microscope.

The porosity of the membrane were measured manually by calculating volume of a porous filler (1-octanol) relative to the total volume of the membrane. The membrane was dried in vacuum at 50°C, and its weight before immersion in 1-octanol was measured as m₀ while irradiating with ultrasound for 5 minutes to fill the pores of the membrane with 1-octanol. The membrane was then taken out, excess 1-octanol was wiped away, and its weight was measured again as m. The porosity δ of the membrane was calculated by the following Expression 3.
[Expression 3] $δ = \frac{4 \left(m-{m}_{0}\right)}{πρ ⋅ \left({D}_{out}^{2}-{D}_{in}^{2}\right) l}$ wherein, *ρ* represents density of 1-octanol, Dₒᵤₜ and Dᵢₙ represent outer and inner diameters of the membrane, respectively, and l represents a length of the membrane.

The average pore size was measured using a liquid-liquid accelerator (LLP-1100A, manufactured by Porous Material Inc., Ithaca, USA). The membrane was cut to a length of 8 cm, and both ends were sealed with epoxy adhesive. Then, the membrane was immersed in IPA (isopropanol) for the whole day and night. Next, using Galwick (propylene, 1, 1, 2, 3, 3, 3 hexafluoride oxide), the IPA was replaced under pressure. Flow velocities under different pressures were recorded by a mass balance for measurement.

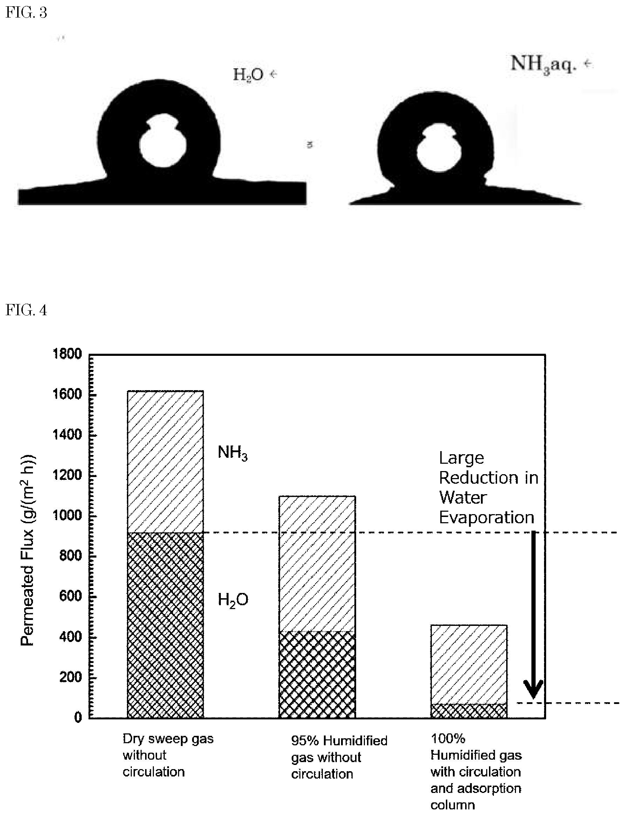

The water contact angle and ammonia water contact angle were measured using a device called DropMaster 300 of Kyowa Interface Science Co., Ltd. FIG. 3 shows microscopic images of the side view.

### (Working Example 1)

The above ammonia adsorption experiment was conducted on the obtained separation membrane (hollow fiber membrane) in the following order: dry sweep gas without circulation, 95% humidified gas without circulation, and 100% humidified gas with circulation and adsorption column. The results are shown in a graph in FIG. 4. While permeation flux was 1600 g/(m²h) in a case of the dry sweep gas without circulation, the permeation flux decreased to approximately 450 g/(m²h), which is less than one-third, in a case of the 100% humidified gas with circulation and adsorption column. The selectivity for ammonia, on the other hand, was greatly improved and this enables to reduce water evaporation significantly. This indicates that the adsorbent selectively adsorbs ammonia. Furthermore, the significant reduction in water evaporation indicates that energy required for water evaporation has also been greatly reduced. The adsorption column referred to here is the column (adsorption portion) made of the copper-iron cyano complex adsorbent obtained in Preparation Example 1.

### (Working Example 2)

The copper-iron cyano complex obtained in Preparation Example 1 was introduced as an adsorbent into the separator 1 in FIG. 1, in which the above-mentioned separation membrane was installed, and separation/concentration of ammonia was performed. That is, a method combining gas sweep membrane distillation method (SGMD) with NH₃ gas adsorbent was employed. Specifically, a dilute ammonia aqueous solution was introduced from the inlet 4 on an one end of the separator 1, in which the hollow fiber membrane made of PVDF was introduced. An excess portion of the dilute ammonia aqueous solution was to be discharged and recovered from the outlet 5 on the other end. On the other end of the separator 1, the inlet 3 for ammonia-containing saturated water (water vapor) was provided, and the saturated water was fed from here via the piping 6a. The saturated water passed though the separator takes in vaporized ammonia components, and was recovered via the piping 6b from the outlet 2 for ammonia-containing saturated water and sent to the adsorbent 7. The adsorbent 7 adsorbed the ammonia and water, and the excess saturated water was sent again to the piping 6a and fed to the separator 1. Such cycle was repeated a predetermined number of times such that the adsorbent 7 adsorbed ammonia and water.

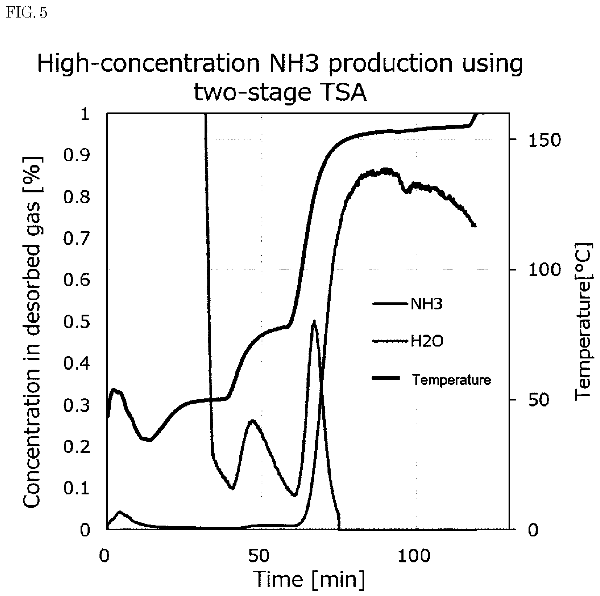

The adsorbent 7, which had adsorbed the ammonia and water, was sent to a heating device, in which its temperature was increased gradually from room temperature. Water and ammonia desorption profiles of the copper-iron cyano complex were checked in advance and are shown in FIG. 5. Below approximately 100°C or less, water is the primary substance that is desorbed. After that, when the temperature reaches 120 to 130°C, ammonia starts to be desorbed. Using such the properties, the adsorbent 7 was treated by heating at 100°C for the first hour and by maintaining the temperature at 150°C for the following hour. This allowed ammonia, which is the volatile gas, to be separated and concentrated up to 96 mass%.

### (Preparation Example 2)

### (Preparation of Manganese-Cobalt Cyano Complex)

Adsorbent powder was prepared by the method described in WO2024048667A1. 20 mL of 0.6 mol/L manganese chloride aqueous solution and 20 mL of 0.3 mol/L potassium hexacyano cobaltate aqueous solution were mixed and shaken for one hour to obtain a precipitate. The liquid containing the precipitate was subjected to solid-liquid separation using a centrifugal force of 16,000 G for ten minutes, and then the supernatant was removed. After adding ultrapure water to the precipitate, the container was covered and shaken up and down to stir the precipitate in the ultrapure water. Such the series of processes, i.e. the centrifugation, removal of the supernatant, and addition of ultrapure water followed by stirring, was repeated six more times. Then, the supernatant was removed and the precipitate was vacuum-dried at room temperature for four hours to obtain powder of manganese-cobalt cyano complex.

### (Working Example 3)

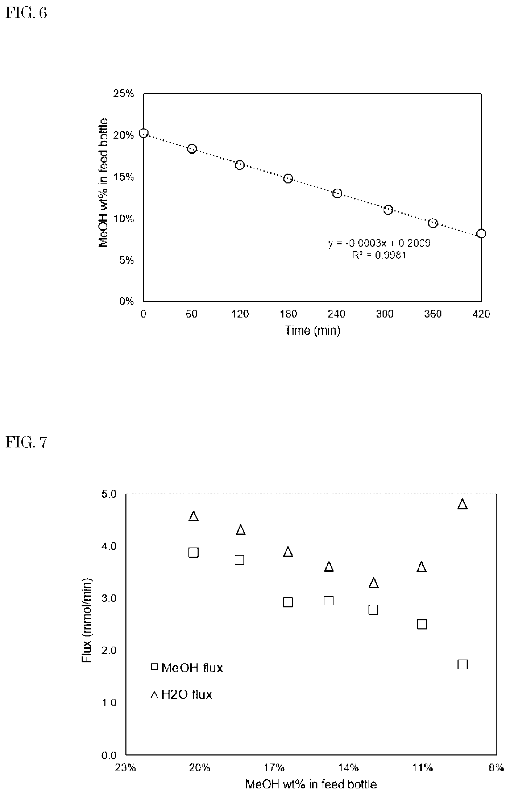

Using the same separation membrane as in Working Example 1, methanol solutions with different concentrations were prepared and a tolerable concentration range was tested, where it was found that the membrane had wetting resistance with methanol solutions at concentrations of 30 vol% or less. Thus, a separation test was conducted using a 24.3 vol% methanol solution, circulating the liquid at 50 ml/min at 27.8°C, and graphs shown in FIG. 6 and FIG. 7 were obtained. It was found, from the graph in FIG. 6, that the concentration of methanol in the circulated methanol solution decreased as methanol was separated and dropped from 14.3 vol% to 8.16 vol% after 420 minutes. The separated methanol was obtained as a gas of 30,000 ppmv maximum, and FIG. 7 shows the gas of 13,500 ppmv when the concentration of the circulating methanol solution was 8.16 vol%.

### (Working Example 4)

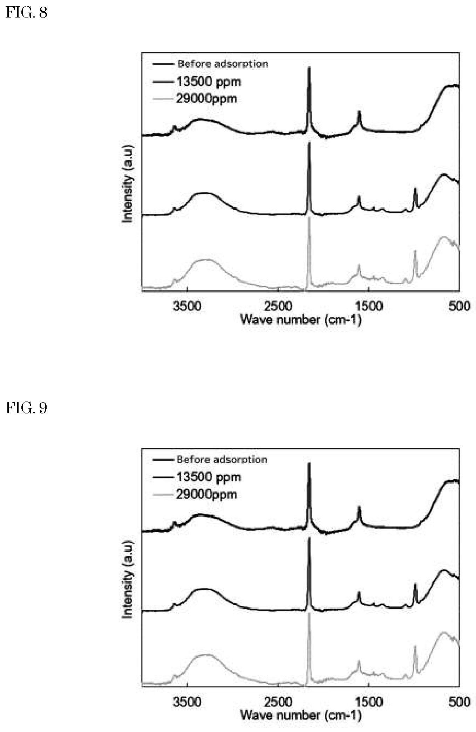

The manganese-cobalt cyano complex obtained in Preparation Example 2 was used as an adsorbent, and methanol adsorption was evaluated at 28 °C with a saturated water vapor amount set at humidity of 100%. The methanol gas concentration was obtained by batch adsorption of methanol at concentrations of 30,000 ppmv and 13,500 ppmv, as obtained in Additional Working Example 1. Methanol solutions and adsorbents were placed in desiccators and left to stand for three days to allow methanol to be adsorbed so as to achieve concentrations of 30,000 ppmv and 13,500 ppmv, respectively. After batch adsorption, the methanol gas concentrations were confirmed to be 29,000 ppmv and 13,500 ppmv using gas detection tubes, and the water vapor concentration was approximately 39,000 ppmv, indicating nearly 100% humidity. FIG. 8 shows infrared spectral diagrams of the manganese-cobalt cyano complex before adsorption and after adsorption under methanol atmosphere of 13,500 ppmv and 29,000 ppmv. Comparing the spectra before and after adsorption, presence or absence of peaks derived from methanol can be seen at approximately 990 cm⁻¹, indicating that 7.1 mmol/g and 9.9 mmol/g of methanol were adsorbed, respectively.

### (Working Example 5)

Methanol and water were separated by heating the adsorbent, which was obtained in Working Example 3, that had adsorbed methanol. FIG. 9 shows infrared spectral diagrams of the manganese-cobalt cyano complex that had adsorbed methanol at 13,500 ppmv when heated. When heated, a water-derived peak observed around 3,300 cm⁻¹ completely disappears at 100°C. Meanwhile, the peak around 990 cm⁻¹, which is derived from methanol, remains. This peak disappears completely by further heating at 150°C, and thus it can be seen that methanol can be concentrated, by using the adsorbent, from the gas containing water vapor and methanol obtained through the separation membrane.

From the results of the above-mentioned working examples, it can be seen that even when an adsorbent made of a Prussian blue (PB) derivative is incorporated into the separation/concentration apparatus of the present invention, excellent selective adsorption and selective desorption properties can be exhibited, and it is possible to efficiently separate and concentrate water-soluble and volatile trace substances from water.

### DESCRIPTION OF NOTATIONS

- 1: membrane distillation portion (separator)
- 2: outlet for gas containing target volatile substance
- 3: inlet for gas after adsorption of target volatile substance
- 4: outlet for dilute ammonia water
- 5: outlet for dilute ammonia water
- 6a: piping (circulation connection portion)
- 6b: piping (circulation connection portion)
- 7: adsorption portion (adsorbent, Prussian blue derivative)
- 10: system for producing a concentration/separation product of ammonia

## Claims

1. A method for producing a separation/concentration product of a volatile substance, which is a method for concentrating an aqueous solution containing a volatile substance or for isolating the volatile substance, the method comprising steps below, and **characterized in that** the volatile substance is selectively adsorbed in an adsorption step below and/or selectively desorbed in a desorption step below;
Step 1: a step for obtaining a mixed gas by using a membrane that is impermeable to water but permeable to gas for separation, with one side of the membrane being a liquid phase side with which the aqueous solution containing the volatile substance comes into contact, and the other side of the membrane being a gas phase side, and allowing the vaporized volatile substance and water vapor to permeate to the gas phase side (Vaporization Step),
Step 2: a step for adsorbing the volatile substance by bringing the gas obtained in Step 1 containing the volatile substance into contact with an adsorbent that can adsorb the volatile substance (Adsorption Step), and
Step 3: a step for desorbing the volatile substance from the adsorbent, obtained in Step 2, that has adsorbed the volatile substance (Desorption Step).

2. The method for producing a separation/concentration product of a volatile substance according to claim 1, wherein
the mixed gas obtained in Step 1 is brought into contact with the adsorbent in Step 2 and, at the same time, the gas after contact is reintroduced to the gas phase side of the membrane in Step 1 so as to suppress vaporization of water.

3. The method for producing a separation/concentration product of a volatile substance according to claim 1, wherein
the volatile substance is ammonia.

4. The method for producing a separation/concentration product of a volatile substance according to claim 1, wherein
the volatile substance is methanol.

5. The method for producing a separation/concentration product of a volatile substance according to claim 1, wherein
the adsorbent is a Prussian blue derivative represented by the following general formula (1):
AₓM[M'(CN)₆]_{y}·zH₂O ··· (1)
wherein x represents a number from 0 to 3, y represents a number from 0.1 to 1.5, z represents a number from 0 to 6, A represents a cation representing at least one or a combination of two or more selected from a group consisting of hydrogen, ammonium cations, alkali metal ions, and alkaline earth metal ions, M and M' are respectively and independently selected, M represents a cation representing at least one or a combination of two or more selected from a group consisting of atoms with atomic numbers from 3 to 83 (excluding ammonium cations, alkali metal ions, and alkaline earth metal ions), and M' represents a cation representing at least one or a combination of two or more selected from a group consisting of atoms with atomic numbers from 3 to 83 (excluding ammonium cations, alkali metal ions, and alkaline earth metal ions).

6. The method for producing a separation/concentration product of a volatile substance according to claim 1, wherein
the volatile substance is desorbed in Desorption Step by heating the adsorbent.

7. The method for producing a separation/concentration product of a volatile substance according to claim 1, wherein
when heating the adsorbent in Desorption Step, temperature is adjusted in two stages, low and high, so that the volatile substance can be selectively desorbed at the high temperature.

8. A system for producing a separation/concentration product of a volatile substance, which is a system for concentrating an aqueous solution containing a volatile substance or for isolating the volatile substance, in which adsorption means below selectively adsorbs the volatile substance and/or desorption means below selectively desorbs the volatile substance, the system comprising:
Means 1: Means for obtaining a mixed gas by using a membrane that is impermeable to water but permeable to gas for separation, with one side of the membrane being a liquid phase side with which the aqueous solution containing the volatile substance comes into contact and the other side of the membrane being a gas phase side, and allowing the vaporized volatile substance and water vapor to permeate to the gas phase side (Vaporization Means),
Means 2: Means for adsorbing the volatile substance by bringing the gas obtained by Means 1 containing the volatile substance into contact with an adsorbent that can adsorb the volatile substance (Adsorption Means), and
Means 3: Means for desorbing the volatile substance from the adsorbent, obtained by Means 2, that adsorbed the volatile substance (Desorption Means).

9. The system for producing a separation/concentration product of a volatile substance according to claim 8, wherein
the adsorbent is a Prussian blue derivative represented by the following general formula (1):
AₓM[M'(CN)₆]_{y} ·zH₂O ... (1)
wherein, x represents a number from 0 to 3, y represents a number from 0.1 to 1.5, z is a number from 0 to 6, A represents a cation representing at least one or a combination of two or more selected from a group consisting of hydrogen, ammonium cations, alkali metal ions, and alkaline earth metal ions, M and M' are respectively and independently selected, M represents a cation representing at least one or a combination of two or more selected from a group consisting of atoms with atomic numbers from 3 to 83 (excluding ammonium cations, alkali metal ions, and alkaline earth metal ions), and M' represents a cation representing at least one or a combination of two or more selected from a group consisting of atoms with atomic numbers from 3 to 83 (excluding ammonium cations, alkali metal ions, and alkaline earth metal ions).

10. The system for producing a separation/concentration product of a volatile substance according to claim 8 further comprising:
means 2.5 (Return Means) provided between means 2 and 3, for returning the gas that is not adsorbed by the adsorbent to the gas phase side of the membrane, thereby enabling continuous vaporization via the membrane.

11. The system for producing a separation/concentration product of a volatile substance according to claim 8, wherein
by Means 3, while the adsorbent obtained by Means 2, which has adsorbed the volatile substance, is removed from a reaction system and the volatile substance is desorbed through a predetermined method, a new adsorbent is supplied to the part from which the adsorbent was removed, thereby maintaining a continuous cycle of vaporization of the volatile substance.
